# EUROPÄISCHE PATENTSCHRIFT

(11) **EP 3 038 509 B1**
(45) Veröffentlichungstag und Bekanntmachung des Hinweises auf die Patenterteilung: **25.12.2019**
(21) Anmeldenummer: 14780403.3
(22) Anmeldetag: 29.08.2014
(51) Int. Cl.: A61B 1/018

(54) **FÜHRUNGSDRAHTHALTER ZUM AUFNEHMEN UND FESTHALTEN EINES MEDIZINISCHEN FÜHRUNGSDRAHTES UND ZUM ANBRINGEN AN EINEM MEDIZINISCHEN GERÄT, INSBESONDERE AN EINEM ENDOSKOP**
GUIDE WIRE HOLDER FOR RECEIVING AND HOLDING A MEDICAL GUIDE WIRE AND FOR ATTACHING SAME TO A MEDICAL DEVICE, IN PARTICULAR AN ENDOSCOPE
PORTE-FIL GUIDE DE RÉCEPTION ET DE MAINTIEN FERME D'UN FIL GUIDE MÉDICAL, À APPLIQUER SUR UN APPAREIL MÉDICAL, EN PARTICULIER UN ENDOSCOPE

(30) Priorität: 30.08.2013 DE 202013007740 U
(43) Veröffentlichungstag der Anmeldung: 06.07.2016
(73) Patentinhaber: Medi-Globe GmbH, 83101 Rohrdorf-Achenmühle (DE)
(72) Erfinder: EDEN, Ingo, 81479 München (DE)
(74) Vertreter: Hofstetter, Schurack & Partner
(86) Internationale Anmeldenummer: PCT/DE2014/000445
(87) Internationale Veröffentlichungsnummer: WO 2015/027979

(56) Entgegenhaltungen:
- DE-A1-102010 011 222
- US-A1- 2004 162 465
- US-A1- 2006 195 117

## Beschreibung

Die Erfindung bezieht sich auf einen Führungsdrahthalter zum Aufnehmen und Festhalten eines eine Eigenelastizität besitzenden medizinischen Führungsdrahtes und zum Anbringen an einem medizinischen Gerät, insbesondere an einem Endoskop, mit einem Anbringungsteil, durch welches der Führungsdraht hindurchführbar ist, und einem mit dem Anbringungsteil verbundenen und von diesem abstehenden Führungsdrahtträger, der den Führungsdraht mittels einer Führungsdraht-Aufnahmeeinrichtung festzuhalten gestattet, die ein erstes, den Führungsdraht in Abstand von dem Anbringungsteil in eine von seiner Durchführungsrichtung in dem Anbringungsteil wegführende Richtung umleitendes und spannendes Führungsdraht-Umlenkglied enthält, das einen auf einem flachen Anfangsbereich des Führungsdrahtträgers sich erhebenden, einseitig offenen Umlenk- und Spannbügel aufweist, welcher den Führungsdraht in einer Öffnung aufzunehmen und aus dieser wieder freizugeben gestattet, und an das sich in weiteren Abständen von dem Anbringungsteil ein zusätzliches Führungsdraht-Umlenkelement und ein noch weiteres Führungsdraht-Umlenkelement anschließen.

Ein Führungsdrahthalter der vorstehend genannten Art ist bereits bekannt (DE 10 2010 011 222 A1; WO 2011/110152 A1). Bei einem konkreten Ausführungsbeispiel dieses bekannten Führungsdrahthalters ist der einseitig offene Umlenk- und Spannbügel durch einen einarmigen Bügel gebildet, der mit einem einzigen Tragteil von dem flachen Anfangsbereich des Führungsdrahthalters absteht und der auf der dem Tragteil gegenüberliegenden Bügelseite offen ist. Grundsätzlich kann jedoch die einseitige Öffnung des Umlenk- oder Spannbügels auch an einer anderen Stelle des Umlenk- oder Spannbügels vorgesehen sein, wie beispielsweise an dessen Oberseite. In diesem Fall wird der Umlenk- und Spannbügel dann zwei Bügelarme enthalten, zwischen denen sich die erwähnte Öffnung befindet. Dabei müssen jedoch besondere Vorkehrungen getroffen werden, um ein sicheres Aufnehmen und Festhalten des Führungsdrahtes unter einem solchen Umlenk- und Spannbügel zu gewährleisten.

Der vorliegenden Erfindung liegt daher die Aufgabe zu Grunde, einen Weg zu zeigen, wie bei einem Führungsdrahthalter der eingangs genannten Art ein solches sicheres Aufnehmen und Festhalten des Führungsdrahtes unter einem zweiarmigen Umlenk- und Spannbügel gewährleistet werden kann.

Gelöst wird die vorstehend aufgezeigte Aufgabe bei einem Führungsdrahthalter der eingangs genannten Art erfindungsgemäß dadurch, dass der Umlenk- und Spannbügel zwei Bügelteile aufweist, die von dem Anfangsbereich des Führungsdrahtträgers abstehen und zwischen denen ein die genannte Öffnung bildender Schlitz vorgesehen ist, der bei elastisch auseinander drückbaren Bügelteilen eine unterhalb der Querabmessung des Führungsdrahtes liegende Schlitzweite und bei starren Bügelteilen eine der Querabmessung des Führungsdrahtes entsprechende Schlitzweite aufweist und der sich zwischen den zwei Bügelteilen in einer Position und/oder Ausrichtung außerhalb einer durch die Eigenelastizität des Führungsdrahts gegebenen Führungsdraht-Aufweitungsbahn von dem Führungsdrahtträger befindet, in der der Führungsdraht versucht, sich in einer Richtung im Wesentlichen senkrecht zu seiner Verlaufsbahn aufzuweiten, indem er gegen die dem Führungsdrahtträger zugewandte Unterseite des Umlenk- und Spannbügels bzw. dessen Bügelteile auf seiner Aufweitungsbahn drückt.

Die Erfindung bringt den Vorteil mit sich, dass durch die Festlegung des Schlitzes in einer Position und/oder Ausrichtung außerhalb der durch die Eigenelastizität des Führungsdrahts gegebenen Führungsdraht-Aufweitungsbahn von dem Führungsdrahtträger weg das sichere Aufnehmen und Festhalten des Führungsdrahtes unter dem Umlenk- und Spannbügel erreicht wird. So kann sich der betreffende Schlitz von der Bügelmitte entfernt in einer Position innerhalb eines der beiden Bügelteile befinden und dabei parallel zur Längsrichtung des Führungsdrahtes verlaufen. Dieser Verlauf kann auch dann gegeben sein, wenn die beiden Bügelteile elastisch auseinander drückbar sind und der Schlitz eine unterhalb der Querabmessung des Führungsdrahtes liegende Schlitzweite aufweist. In dem Fall, dass sich der genannte Schlitz in einer Position im Bereich der Bügelmitte befindet, wird er vorzugsweise allerdings quer zur Längsrichtung des Führungsdrahtes ausgerichtet sein. Diese Querausrichtung des Schlitzes kann jedoch dann vorgesehen sein, wenn sich dieser in einer Position außerhalb des Bereiches der Bügelmitte befindet.

Im Zusammenhang mit dem erwähnten Führungsdraht sei hier noch folgendes angemerkt. Unter der erwähnten Führungsdraht-Aufweitungsbahn wird hier die Bahn des Führungsdrahtes verstanden, in deren Richtung der an zwei Fixierungspunkten fixierte Führungsdraht versucht, sich auf Grund seiner Eigenelastizität aufzuweiten. Im vorliegenden Fall sind die beiden Fixierungspunkte des Führungsdrahtes auf dessen Verlaufsbahn zwischen dessen Anlegepunkten bzw. -flächen an der Austrittsstelle aus der Abdeckplatte des Anbringungsteiles und an dem Umlenk- und Spannbügel gegeben. Auf dieser Verlaufsbahn versucht der eigenelastische Führungsdraht sich in einer Richtung im Wesentlichen senkrecht zu dieser Verlaufsbahn mit der Folge aufzuweiten, dass er gegen die dem Führungsdrahtträger zugewandte Unterseite des Umlenk- und Spannbügels bzw. dessen Bügelteile auf seiner Aufweitungsbahn drückt. Unter der Querabmessung des Führungsdrahtes wird generell dessen Abmessung verstanden, die quer, also um 90° zu dessen Längsachse verläuft; im Falle eines einen runden Querschnitt aufweisenden Führungsdrahts ist die betreffende Querabmessung durch den Durchmesser des betreffenden Führungsdrahts gegeben. Besitzt der verwendete Führungsdraht einen viereckigen Querschnitt mit unterschiedlichen Kantenlängen, so ist unter dessen Querabmessung die eine Kantenlänge dieses viereckigen Querschnitts zu verstehen, die die kürzeste Kantenlänge aufweist. Im Falle eines ovalen Querschnitts mit einem großen Durchmesser und einem demgegenüber kleinen Durchmesser ist unter der Querabmessung des Führungsdrahtes dessen kleiner Durchmesser zu verstehen.

Gemäß einer zweckmäßigen Weiterbildung der Erfindung weist eines der beiden Bügelteile ein von dem Anfangsbereich des Führungsdrahtträgers abstehendes Tragglied und ein von diesem getragenes Oberteil mit einem gegenüber dem Querschnitt des Tragteiles ausladenden Querschnitt bzw. Körpervolumen auf, und das andere Bügelteil ist lediglich ein Tragteil, das dem genannten Oberteil unter Bildung des Schlitzes in Abstand benachbart ist. Hierdurch ergibt sich der Vorteil, dass mit einer relativ einfachen Bügelkonstruktion ausgekommen werden kann.

Vorzugsweise ist der Schlitz lediglich im Bereich des ausladenden Querschnitts bzw. Körpervolumens des Oberteiles des einen Bügelteiles und dem das andere Bügelteil bildenden Tragteil vorgesehen. Dadurch kann auf besonders einfache Weise - bei Betrachtung des Führungsdrahthalters von der Seite des ausladenden Querschnitts bzw. Körpervolumens des Oberteiles des einen Bügelteiles aus - unterhalb des genannten Schlitzes ein zuweilen ausreichender Raum zum sicheren Aufnehmen und Festhalten eines durch den erwähnten Schlitz eingeführten Führungsdrahtes bereitgestellt werden.

Gemäß einer anderen zweckmäßigen Weiterbildung der Erfindung weist jedes der beiden Bügelteile ein von dem Anfangsbereich des Führungsdrahtträgers abstehendes Tragglied und ein von diesem getragenes Oberteil mit einem gegenüber dem Querschnitt des zugehörigen Tragteiles ausladenden Querschnitt bzw. Körpervolumen auf, und die Oberteile der beiden Bügelteile sind unter Bildung des genannten Schlitzes voneinander in Abstand vorgesehen. Hierdurch ergibt sich ebenfalls der Vorteil einer relativ einfachen Bügelkonstruktion, die zudem mit zumindest weitgehend symmetrisch zu gestaltenden Bügelteilen realisierbar ist.

Vorzugsweise ist bei den beiden zuletzt betrachteten zweckmäßigen Weiterbildungen der Erfindung das jeweilige Tragteil, welches ein Oberteil trägt, mit seiner dem Schlitz zugewandten Seite von diesem zurück versetzt. Hierdurch ergibt sich der Vorteil, dass - bei Betrachtung des Führungsdrahthalters von der Seite der ausladenden Querschnitte bzw. Körpervolumen der Oberteile beider Bügelteile aus - unterhalb des genannten Schlitzes ein relativ großer Raum zum sicheren Aufnehmen und Festhalten eines durch den erwähnten Schlitz eingeführten Führungsdrahtes bereitgestellt werden kann.

Gemäß einer noch anderen zweckmäßigen Weiterbildung der Erfindung ist das jeweilige Oberteil mit seinem gegenüber dem Querschnitt seines Tragteiles ausladenden Querschnitt bzw. Körpervolumen rollen-, walzen-, kegel-, tonnen- oder kugelförmig ausgebildet. Dadurch lassen sich für ein Einführen und Festhalten eines Führungsdrahtes in die Bügelanordnung besonders wirksame Bügelteile realisieren.

Vorzugsweise bildet das jeweilige Oberteil zu dem Schlitz hin eine abgeflachte Fläche. Diese Maßnahme bringt den Vorteil mit sich, dass der betreffende Schlitz ein Herausgleiten des Führungsdrahtes aus dem Raum, der sich unterhalb des Oberteils des jeweiligen mit einem gegenüber dem Querschnitt des zugehörigen Tragteiles ausladenden Querschnitte bzw. Körpervolumen befindet, wirksam und damit besser verhindert oder zumindest erschwert als ein punkt- bzw. linienförmigen gestalteter Schlitz. Außerdem kann die abgeflachte Fläche des Schlitzes - bei Betrachtung des Führungsdrahthalters in Richtung der abgeflachten Fläche eine von der Vertikalen abweichende Neigung besitzen.

Zweckmäßigerweise weist der Schlitz einen geradlinigen Verlauf auf. Dies bringt den Vorteil mit sich, dass sich der betreffende Schlitz besonders einfach herstellen lässt.

Vorzugsweise verläuft der im mittleren Bereich des Umlenk- und Spannbügels zwischen dessen beiden Bügelteilen vorgesehene Schlitz unter einem Winkel von etwa 5° bis etwa 175° bezogen auf die Aufnahme- und Festhaltebahn des Führungsdrahts auf dem Führungsdrahtträger. Innerhalb dieses Winkelbereichs ist ein Herausgleiten des Führungsdrahtes aus dem Raum, der sich unterhalb der Oberteile der jeweiligen mit einem gegenüber dem Querschnitt des zugehörigen Tragteiles ausladenden Querschnitten bzw. Körpervolumen befindet, besonders wirksam verhindert.

Von Vorteil ist es ferner, wenn der Führungsdrahtträger im Bereich der Aufnahme- und Festhaltebahn des Führungsdrahts ein Material, wie Gummi oder Silikon aufweist, welches auf den daran anliegenden Führungsdraht einen Reibwiderstand ausübt. Dadurch ist ein sicheres Festhalten des Führungsdrahts in dem Raum, der sich unterhalb des Oberteils des jeweiligen mit einem gegenüber dem Querschnitt des zugehörigen Tragteiles ausladenden Querschnitt bzw. Körpervolumens befindet, ganz besonders wirksam.

An Hand von Zeichnungen wird die Erfindung nachstehend an Ausführungsbeispielen näher erläutert.

In den Zeichnungen zeigen
- Fig. 1: eine schematische Seitenansicht eines Führungsdrahthalters gemäß einer Ausführungsform der vorliegenden Erfindung in einer Größe, die von der in der Praxis verwendeten Größe verschieden sein kann,
- Fig. 2: eine Perspektivansicht des in Fig. 1 gezeigten Führungsdrahthalters von schräg oben aus betrachtet,
- Fig. 3: eine Draufsicht des in dem eingangs genannten Dokument (DE 10 2010 011 222 A1; WO 2011/110152 A1) angegebenen Führungsdrahthalters mit einem Führungsdrahthalter gemäß einer Ausführungsform der vorliegenden Erfindung,
- Fig. 4: eine Schnittansicht längs der in Fig. 3 eingetragenen Schnittebene II - II,
- Fig. 5: eine ausschnittweise Draufsicht auf den in Fig. 1 dargestellten Führungsdrahthalter in der dort durch einen Pfeil angegebenen Betrachtungsrichtung A,
- Fig. 6: eine ausschnittweise Perspektivansicht eines Führungsdrahthalters gemäß einer anderen Ausführungsform der Erfindung in einer Größe, die von der in der Praxis verwendeten Größe verschieden sein kann, und
- Fig. 7: eine ausschnittweise Perspektivansicht eines Führungsdrahthalters gemäß einer noch weiteren Ausführungsform der Erfindung in einer Größe, die von der in der Praxis verwendeten Größe verschieden sein kann.

Bevor auf die Zeichnungen näher eingegangen wird, sei angemerkt, dass in sämtlichen Zeichnungen gleiche oder einander entsprechende Einrichtungen bzw. Elemente grundsätzlich mit gleichen Bezugszeichen bezeichnet sind.

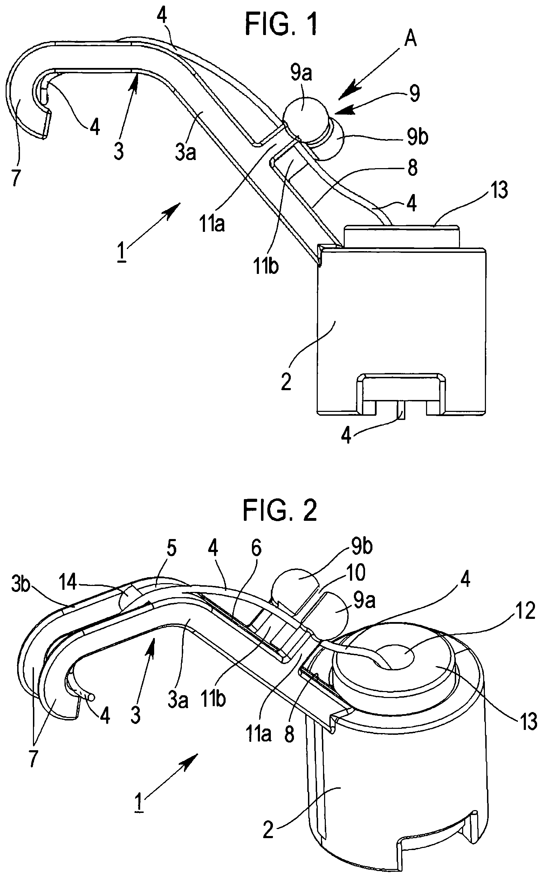

In Fig. 1 ist ein Führungsdrahthalter 1 gemäß einer Ausführungsform der vorliegenden Erfindung in einer Seitenansicht dargestellt. Dieser Führungsdrahthalter 1 enthält wie der in dem eingangs genannten Dokument angegebene Führungsdrahthalter ein Anbringungsteil 2 zum Anbringen des Führungsdrahthalters 1 an einem (hier nicht dargestellten) medizinischen Gerät, welches insbesondere ein Endoskop sein kann, an dessen Arbeitskanalanschluss das betreffende Anbringungsteil 2 angebracht werden kann. Das Anbringungsteil 2 kann auch hier zum Beispiel ein Aufsetzteil oder eine Kappe sein, die auf ein Aufnahmeteil des jeweiligen medizinischen Geräts aufsetzbar ist und dort nicht leicht abziehbar ist. Das betreffende Anbringungsteil 2 kann aber gegebenenfalls auch ein Schraubverbindungsteil enthalten, mit dem es auf oder in einen entsprechenden Schraubverbindungsteil des jeweiligen medizinischen Geräts verschraubbar ist.

Mit dem Anbringungsteil 2 ist ein Führungsdrahtträger 3 verbunden, der einen eine Eigenelastizität aufweisenden medizinischen Führungsdraht 4 mittels einer Führungsdraht-Aufnahmeeinrichtung festzuhalten gestattet. Der Führungsdrahtträger 3 enthält, wie dies aus der Perspektivdarstellung gemäß Fig. 2 und der Draufsicht gemäß 3 deutlich ersichtlich ist, zwei miteinander verbundene Trägerarme 3a und 3b, die in einen Anfangsbereich 8 zwischen ihrer Verbindungsstelle mit dem Anbringungsteil 2 und einer Abschlussstelle 5 durch einen vorzugsweise flachen bzw. geradlinig verlaufenden Verbindungsteil 6 miteinander verbunden sind.

Der Führungsdrahtträger 3 und damit dessen beide Trägerarme 3a und 3b weisen bei Betrachtung in der Seitenansicht gemäß Fig. 1 einen schräg nach links oben gerichteten Verlauf auf. Nach Erreichen einer bestimmten festgelegten Höhe bei der erwähnten Abschlussstelle 5 verlaufen die beiden Trägerarme 3a und 3b gemäß Fig. 1 und 2 zuerst etwa waagerecht und dann nach unten, also zur Ebene des Anbringungsteiles 2 hin abgebogen, um schließlich an ihrem jeweiligen Ende in einem gebogenen Kröpfungsbereich 7 auszulaufen.

Die Abschlussstelle 5 ist gemäß Fig. 1 und 2 durch einen unterhalb der in diesen Figuren oben dargestellten Oberseite des Führungsdrahtträgers 3 liegenden Trägerbereich gebildet, in welchem der Führungsdraht 4 aufnehmbar ist. Die Absenkung der Führungsbahn von der Oberseite des Führungsdrahtträgers 3 liegt beispielsweise zwischen 0,5mm und 4mm.

Die Führungsdraht-Aufnahmeeinrichtung des Führungsdrahtträgers 3 gestattet es, den Führungsdraht 4 so festzuhalten, dass dieser in seinem aufgenommenen Zustand nicht in seiner Längsrichtung verschoben bzw. gezogen werden kann. Dazu umfasst die Führungsdraht-Aufnahmeeinrichtung zum einen von dem Anbringungsteil 2 aus ein auf dem flachen Anfangsbereich 8 des Führungsdrahtträgers 3 sich erhebendes erstes Führungsdraht-Umlenkglied 9, das als einseitig offener Umlenk- und Spannbügel 9 ausgebildet ist. Dieser Umlenk- und Spannbügel 9 besteht aus zwei Bügelteilen 9a und 9b, die von dem Anfangsbereich 8 des Führungsdrahtträgers 3 abstehen und zwischen denen ein die genannte eine Öffnung bildender Schlitz 10 vorgesehen ist, der hier eine der Querabmessung d des Führungsdrahtes 4 entsprechende Schlitzweite w aufweist und der sich hier zwischen den zwei Bügelteilen 9a, 9b in einer Position und/oder Ausrichtung außerhalb einer durch die Eigenelastizität des Führungsdrahts 4 gegebenen Führungsdraht-Aufweitungsbahn von dem Führungsdrahtträger 3 befindet. Die der Querabmessung d des Führungsdrahtes 4 entsprechende Schlitzweite w ist bei starren Bügelteilen 9a, 9b vorzugsweise gleich groß wie die betreffende Querabmessung d; sie kann aber auch größer sein als diese Querabmessung d.

Wie oben bereits erwähnt, wird hier unter der erwähnten Führungsdraht-Aufweitungsbahn diejenige Bahn des Führungsdrahtes 4 verstanden, in deren Richtung der an zwei Fixierungspunkten fixierte Führungsdraht 4 versucht, sich auf Grund seiner Eigenelastizität aufzuweiten. Im vorliegenden Fall sind diese beiden Fixierungspunkte des Führungsdrahtes auf dessen Verlaufsbahn zwischen den Anlegepunkten bzw. -flächen an der Austrittsstelle der Durchgangsöffnung 12 aus der Abdeckplatte 13 des Anbringungsteiles 2 und an dem Umlenk- und Spannbügel 9 gegeben. Auf dieser Verlaufsbahn versucht der eigenelastische Führungsdraht 4 sich in einer Richtung im Wesentlichen senkrecht zu dieser Verlaufsbahn mit der Folge aufzuweiten, dass er gegen die dem Führungsdrahtträger 3 zugewandte Unterseite des Umlenk- und Spannbügels 9 bzw. dessen Bügelteile 9a, 9b auf seiner Aufweitungsbahn drückt.

An dieser Stelle sei noch angemerkt, dass die betreffende Querabmessung d normalerweise durch den Durchmesser eines Führungsdrahts 4 mit rundem Querschnitt gegeben ist. Besitzt der verwendete Führungsdraht indessen einen viereckigen Querschnitt, ist demgegenüber unter der betreffenden Querabmessung die eine Kantenlänge des einen viereckigen Querschnitt aufweisenden Führungsdrahtes 4 zu verstehen, und zwar vorzugsweise dessen kürzere Querschnitts-Kantenlänge bei unterschiedlichen Querschnitts-Kantenlängen. Im Falle eines ovalen Querschnitts mit einem großen Durchmesser und einem demgegenüber kleinen Durchmesser ist unter der Querabmessung des Führungsdrahtes dessen kleiner Durchmesser zu verstehen.

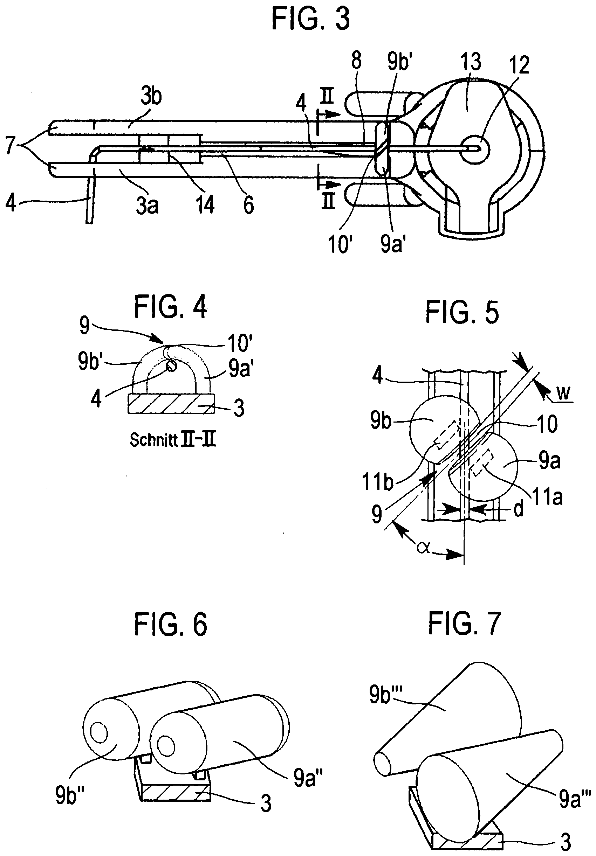

In Fig. 1, 2 und 5 sind die beiden Bügelteile 9a, 9b jeweils durch ein von dem Anfangsbereich 8 des Führungsdrahtträgers 3 abstehendes Tragglied 11a bzw. 11b und ein von diesem getragenes kugelförmiges Oberteil mit einem gegenüber dem Querschnitt des zugehörigen Tragteiles 11a bzw. 11b ausladenden größeren Querschnitt bzw. Körpervolumen gebildet. Die beiden kugelförmigen Oberteile sind hier der Einfachheit halber ebenfalls mit 9a, 9b bezeichnet, also mit denselben Bezugszeichen, die für die beiden Bügelteile verwendet sind - da diese kugelförmigen Oberteile die eigentliche Funktion des Umlenk- und Spannbügels 9 erfüllen. Die Tragteile 11a, 11b sind hier vorzugsweise mit ihren einen, einander gegenüberliegenden Seiten von Rändern des Schlitzes 10 der von ihnen getragenen Oberteile 9a, 9b zurück versetzt, wie dies aus den Darstellungen gemäß Fig. 2 und 5 klar hervorgeht. Dieser Schlitz 10 befindet sich hier in Bezug auf die Bügelteile bzw. Oberteile 9a und 9b somit in der Mittenposition des Umlenk- und Spannbügels 9 in dessen von den Tragarmen 3a und 3b am weitesten weg liegenden Bereich. Die betreffenden Tragteile 11a, 11b können jedoch in Abweichung von den dargestellten Verhältnissen auch relativ kurz ausgebildet sein und gegebenenfalls sogar gänzlich entfallen. In diesem zuletzt erwähnten Fall sind die kugelförmigen Oberteile 9a, 9b dann direkt an dem Führungsdrahtträger 3 angebracht, vorzugsweise mit einem nahezu punktförmigen, jedenfalls mit einem gegenüber dem Oberteilquerschnitt eine sehr kleine Kontaktfläche aufweisenden Verbindungsbereich.

Die kugelförmigen Oberteile 9a, 9b der beiden Bügelteile sind hier unter Bildung des genannten Schlitzes 10 voneinander in Abstand vorgesehen. Dieser Schlitz 10 ist dabei vorzugsweise durch abgeflachte Flächen in den einander gegenüber liegenden Seiten der kugelförmigen Oberteile 9a und 9b gebildet. Dabei weist der Schlitz 10 vorzugsweise einen geradlinigen Verlauf auf, wie dies aus Fig. 5 klar hervorgeht, die einen Ausschnitt des in Fig. 1 dargestellten Führungsdrahthalters 1 in der dort durch einen Pfeil angegebenen Betrachtungsrichtung A zeigt. Überdies lässt Fig. 5 erkennen, dass die Tragteile 11a, 11b mit ihren Außenseiten gegenüber den Außenseiten der von ihnen getragenen im Querschnitt ausladenden Oberteile 9a und 9b zurück versetzt sind. Dadurch kann der Führungsdraht 4 unter den kugelförmigen Bereichen der Oberteile 9a und 9b anliegen, die nicht unmittelbar mit den Tragteilen 11a, 11b verbunden sind. Die betreffenden Tragteile 11a, 11b können im übrigen mit den Oberteilen 9a und 9b auch an solchen Stellen verbunden sein, dass sie mit ihren einander gegenüberliegenden Seiten mit den den Schlitz 10 bildenden Seiten der Oberteile 9a und 9b fluchten. In jedem Fall lässt sich der Führungsdraht 4 so sicher unter den beiden kugelförmigen Oberteilen 9a und 9b halten, wie dies auch aus Fig. 1 und 2 ersichtlich ist.

Generell kann der Schlitz 10 auch eine andere Verlaufsform aufweisen als die dargestellte, also beispielsweise einen bogenförmigen Verlauf. Der Schlitz 10 verläuft hier bei dem aus Fig. 1 und 2 ersichtlichen Aufbau des Umlenk- und Spannbügels 9 unter einem optimalen Winkel α von etwa 45° bezogen auf die Verlaufs- oder Aufnahme- und Festhaltebahn des Führungsdrahts 4 auf dem Führungsdrahtträger 3, das heißt hier in Bezug auf die Längsrichtung des Führungsdrahtes 4, wie dies ebenfalls aus Fig. 5 ersichtlich ist. Generell kann der Winkel α hier zwischen 5° und 175° bezogen auf die Aufnahme- und Festhaltebahn des Führungsdrahts 4 auf dem Führungsdrahtträger 3 bzw. bezogen auf die Längsrichtung des Führungsdrahtes 4 in dem Bereich betragen, in welchem der Führungsdraht 4 auf Grund seiner Eigenelastizität an der Innenseite des Umlenk- und Spannbügels 9 anzuliegen vermag.

Der Eigenelastizität besitzende medizinische Führungsdraht 4 wird zu seiner Aufnahme und zu seinem Festhalten in dem Führungsdrahthalter 1 zunächst an einer oberen Kante einer Durchgangsöffnung 12 einer Abdeckplatte 13 des Anbringungsteiles 2 in Abstand von dem Anbringungsteil 2 in eine von dessen Durchführungsrichtung in dem Anbringungsteil 2 wegführende Richtung umgeleitet und dabei, wie dies aus Fig. 1 und 2 ersichtlich ist, unter den zu den beiden Bügelteilen gehörenden kugelförmigen Oberteilen 9a und 9b gespannt gehalten, bevor er zu der ein zusätzliches bzw. weiteres Führungsdraht-Umlenkelement darstellenden Abschlussstelle 5 zwischen den beiden Trägerarmen 3a und 3b hin gelangt, an der auf den Führungsdraht 4 eine Reibungswirkung ausgeübt wird. Diese Abschlussstelle 5 kann durch ein zusätzliches Führungsdraht-Umlenkelement 14 gebildet sein, das durch eine Führungsdraht-Anlagefläche auf der Oberseite des Führungsdrahtträgers 3 gebildet ist. Auf dieser Führungsdraht-Anlagefläche ist der Führungsdraht 4 längs einer Führungsbahn führbar, durch die im Bereich der betreffenden Führungsdraht-Anlagefläche ein Reibungswiderstand gegenüber dem über die bzw. auf der Oberseite des Führungsdrahtträgers 3 geführten Führungsdraht 4 ausübbar ist.

Das zusätzliche Führungsdraht-Umlenkelement 14 ist hier durch eine einfache gemäß Fig. 1 und 2 zu dem Anbringungsteil 2 hin gerichtete Bogenfläche gebildet, die wie die Aufnahme- und Festhaltebahn des Führungsdrahts 4 ein Material, wie Gummi oder Silikon aufweisen kann, welches auf den daran anliegenden Führungsdraht 4 einen Reibwiderstand ausübt.

Abweichend von dem vorstehend erläuterten Aufbau des zusätzlichen Führungsdraht-Umlenkelements 14 kann dieses durch eine in dem Führungsdrahtträger 3 enthaltene Rolleneinrichtung in Form eines einzelnen Rollengliedes gebildet sein. Dieses Rollenglied kann ein feststehendes Rollenglied oder ein Rollenglied sein, welches nur schwer drehbar ist, wenn der Führungsdraht 4 über dieses Rollenglied gezogen wird. In beiden Fällen kann das jeweilige Rollenglied eine raue Oberfläche besitzen, so dass dem über dieses Rollenglied gezogene Führungsdraht 4 ein Reibungswiderstand entgegengesetzt wird.

Alternativ dazu kann die zuvor erwähnte Rolleneinrichtung durch zwei axial oder radial federnd zusammengeführte Rolleneinrichtungsteile gebildet sein, zwischen denen der Führungsdraht 4 aufnehmbar ist. Auch in diesem Fall können die beiden Rolleneinrichtungsteile jeweils eine raue Oberfläche besitzen, so dass auch hierbei dem über dieses Rollenglied gezogene Führungsdraht 4 ein Reibungswiderstand entgegengesetzt wird.

Die erwähnte Rauigkeit der Führungsdraht-Anlagefläche 9 kann beispielsweise durch eine Strukturierung dieser Fläche und/oder durch einen auf die betreffende Fläche aufgebrachten entsprechend rauen Flächenüberzug erreicht sein.

Zum Arbeiten mit dem Führungsdraht 4 in Verbindung mit einem (nicht dargestellten) medizinischen Gerät, wie einem Endoskop wird der Führungsdraht 4 zunächst durch die Öffnung 12 der Abdeckplatte 13 des auf dem betreffenden medizinischen Gerät aufgesetzten Anbringungsteiles 2 soweit eingeführt, bis die (nicht gezeigte) Spitze des Führungsdrahtes 4 in ein gewünschtes Zielgebiet vorgeschoben worden ist.

Sodann wird der aus der Öffnung 12 der Abdeckplatte 13 des Anbringungsteiles 2 herausragende Abschnitt des Führungsdrahtes 4 nach Hineinführen in das Führungsdraht-Umlenkglied 9 durch den dort vorhandenen Schlitz 10 in die Führungsbahn auf der Oberseite des Führungsdrahtträgers 3 aufgelegt, um mit der genannten einen Abschlussstelle 5 und dem zusätzlichen Umlenkelement 14 in Anlage zu gelangen. Anschließend wird der um dieses Umlenkelement 14 herunter geführte und von dem Anbringungsteil 2 am weitesten weg liegende Teil des Führungsdrahtes 4 um den ein noch weiteres Führungsdraht-Umlenkelement darstellenden Kröpfungsbereich 7 des Führungsdrahtträgers 3 herum gelegt, und zwar um einen der Trägerarme 3a und 3b dieses Führungsdrahtträgers 3 - hier um den Trägerarm 3a. Dadurch wird der Führungsdraht 4 winklig, und zwar um etwa 90° aus seiner Führungsbahn auf der Oberseite des Führungsdrahtträgers 3 umgelenkt, wie dies aus Fig. 2 ersichtlich ist. Der Führungsdraht 4 ist damit in eine gewünschte Richtung gewissermaßen "aufgeräumt", in der er in seiner Längsrichtung auf dem Führungsdrahtträger 3 jetzt nicht mehr verschiebbar ist.

Für diese Nichtverschiebbarkeit des Führungsdrahtes 4 in dessen Längsrichtung auf dem Führungsdrahtträger 3 sind vor allem die Anlagen des Führungsdrahtes 4 an der Austrittsstelle aus der Abdeckplatte 13 des Anbringungsteiles 2, an dem Führungsdraht-Umlenkglied 6 auf der Oberseite des Führungsdrahtträgers 3 und an dem Umlenkelement 14 auf der Oberseite des Führungsdrahtträgers 3 maßgebend. Die Anlagen des Führungsdrahtes 4 an dem Kröpfungsbereich 7 des Führungsdrahtträgers 3 wirkt sich unterstützend auf die zuvor erläuterte Nichtverschiebbarkeit des Führungsdrahtes 4 in dessen Längsrichtung auf dem Führungsdrahtträger 3 aus.

Die beiden Bügelteile 9a, 9b des Führungsdraht-Umlenkgliedes 14 können nun auch anders gestaltet sein als dies zuvor an Hand der Fig. 1, 2 und 5 erläutert worden ist. So kann beispielsweise eines der beiden Bügelteile 9a, 9b ein von dem Anfangsbereich des Führungsdrahtträgers 3 abstehendes Tragglied, wie das Tragglied 11a oder 11b und ein von diesem getragenes Oberteil 9a oder 9b mit einem gegenüber dem Querschnitt des betreffenden Tragteiles ausladenden Querschnitt bzw. Körpervolumen aufweisen, und das andere Bügelteil, also 9b oder 9a kann lediglich ein dem genannten Oberteil benachbartes Tragteil sein, das mit einer Seite im Abstand der Schlitzweite von dem betreffenden Oberteil 9b bzw. 9a des einen der beiden Bügelteile vorgesehen ist.

Der Schlitz 10 mit seiner der Querabmessung d des Führungsdrahtes 4 entsprechenden Schlitzweite w kann in diesem Fall lediglich im Bereich des ausladenden Querschnitts bzw. Körpervolumens des Oberteiles des einen Bügelteiles 9a oder 9b und dem das andere Bügelteil 9b bzw. 9a bildenden Tragteil vorgesehen sein, und gegebenenfalls ist das dieses Oberteil tragende Tragglied mit seiner dem Schlitz zugewandten Seite diesem gegenüber zurück versetzt.

Eine andere Gestaltungsmöglichkeit für die zwei Bügelteile 9a, 9b des Führungsdraht-Umlenkgliedes 9 ist aus Fig. 3 und 4 ersichtlich. Hier sind die beiden Bügelteile 9a', 9b' durch einen beispielsweise runden Querschnitt aufweisende bogenförmige Bügelteile gebildet, die im Bereich ihren von dem Führungsdrahtträger 3 am weitesten abstehenden Stellen durch einen Schlitz 10' voneinander getrennt sind, für dessen Abmessung dasselbe gilt, was zum Schlitz 10 oben ausgeführt worden ist. Der Schlitz 10' verläuft wie der Schlitz 10 schräg (vorzugsweise unter einem Winkel von etwa 45°) zur Längsrichtung des von dem Führungsdrahthalter 1 aufgenommenen Führungsdrahtes 4. Der betreffende Schlitz 10' könnte jedoch auch parallel zur Längsrichtung des von dem Führungsdrahthalter 1 aufgenommenen Führungsdrahtes 4 verlaufen, wenn er in einem Seitenbereich des die beiden Bügelteile 9a' und 9b' umfassenden Bügels vorhanden wäre.

In dem Fall, dass die beiden Bügelteile 9a, 9b oder 9a', 9b' eine solche Elastizität aufweisen, dass sie auseinander gedrückt werden können, wie durch Hindurchdrücken des Führungsdrahtes 4 durch den zwischen ihnen gebildeten Schlitz, könnte überdies der zuvor erwähnte, parallel zur Längsrichtung des von dem Führungsdrahthalter 1 aufgenommenen Führungsdrahtes 4 verlaufende Schlitz auch an den von dem Führungsdrahtträger 3 am weitesten weg befindlichen Stellen gebildet sein. Der betreffende Schlitz könnte in diesem Fall eine Schlitzweite aufweisen, die unterhalb der erwähnten Querabmessung des Führungsdrahtes 4 liegt.

Zwei noch weitere Gestaltungsmöglichkeiten für die zwei Bügelteile 9a, 9b des Führungsdraht-Umlenkgliedes 9 sind in Fig. 6 und 7 dargestellt.

Gemäß Fig. 6 ist das jeweilige Oberteil - hier mit 9a" bzw. 9b" bezeichnet - mit seinem gegenüber dem Querschnitt seines nicht näher bezeichneten Tragteiles ausladenden Querschnitt bzw. Körpervolumen rollen-, walzen- oder tonnenförmig ausgebildet. Zwischen diesen rollen-, walzen- oder tonnenförmigen Oberteilen 9a" bzw. 9b" ist wie zwischen den Bügelteilen 9a und 9b der oben erläuterten Ausführungsbeispiele ein Schlitz vorhanden.

Gemäß Fig. 7 ist das jeweilige Oberteil - hier mit 9a''' bzw. 9b''' bezeichnet - mit seinem gegenüber dem Querschnitt seines nicht näher dargestellten Tragteiles ausladenden Querschnitt bzw. Körpervolumen kegelförmig ausgebildet. Unter "kegelförmig" wird hier sowohl eine Vollkegelform als auch eine Kegelstumpfform verstanden. Auch zwischen diesen kegelförmigen Oberteilen 9a''' bzw. 9b''' ist wie zwischen den Bügelteilen 9a und 9b der oben erläuterten Ausführungsbeispiele ein Schlitz vorhanden.

Bei den Ausführungsformen der Oberteile 9a", 9b" gemäß Fig. 5 und 9a''', 9b''' gemäß Fig. 7 können deren Tragteile jeweils stift- oder zylinderförmig gestaltet sein. Die betreffenden Tragteile können jedoch alternativ dazu auch durch Stegteile gebildet sein, mit denen die Oberteile des jeweiligen Oberteilpaares von ihren gegenüber liegenden Enden aus mit dem Führungsdrahtträger 3 verbunden sind. Diese Stegteile erstrecken sich vorzugsweise höchstens bis zur bzw. kurz vor der Längsmitte des jeweiligen Oberteiles.

Abschließend sei noch angemerkt, dass das Anbringungsteil 2 aus einem biokompatiblen Material, wie einem Metall oder Kunststoff, zum Beispiel aus einem Gummimaterial bestehen kann. Alle übrigen Elemente des hier beschriebenen Führungsdrahthalters 1 bestehen ebenfalls aus einem biokompatiblen Material, wie aus einem Metall oder Kunststoff, wie zum Beispiel aus einem ABS-Kunststoff (Acrylnitril-Butadien-Styrol-Copolymerisat), und zwar vorzugsweise als ein einziges, zusammenhängendes Form- bzw. Spritzgussteil. Dabei sind Kanten von Teilen des Führungsdrahthalters 1, an denen der Führungsdraht 4 entlang geführt ist, vorzugsweise verrundet, um eine Beschädigung der Führungsdrahtoberfläche zu vermeiden und um ein Einlegen des Führungsdrahtes 4 in den Führungsdrahthalter 1 für den Anwender zu vereinfachen.

Der Eigenelastizität aufweisende Führungsdraht 4 besteht, wie an sich bekannt, aus einem Edelstahlkern, der mittels Polytetrafluorethylen - PTFE - (Handelsmarke: Teflon) ummantelt ist.

### Bezugszeichenliste

- 1: Führungsdrahthalter
- 2: Anbringungsteil
- 3: Führungsdrahtträger
- 4: Führungsdraht
- 5: Abschlussstelle
- 6: Verbindungsteil
- 7: Kröpfungsbereich
- 8: Anfangsbereich
- 9: Umlenk- und Spannbügel
- 9a, 9b: Bügelteil, Oberteil
- 9a', 9b': Bügelteil, Oberteil
- 9a", 9b": Bügelteil, Oberteil
- 9a''', 9b''': Bügelteil, Oberteil
- 10, 10': Schlitz
- 11a, 11b: Tragglied
- 12: Durchgangsöffnung
- 13: Abdeckplatte
- 14: Führungsdraht-Umlenkelement, Bogenfläche
- A: Betrachtungsrichtung
- α: Winkel
- d: Querabmessung/Durchmesser des Führungsdrahtes 4
- w: Schlitzweite

## Patentansprüche

1. Führungsdrahthalter (1) zum Aufnehmen und Festhalten eines eine Eigenelastizität besitzenden medizinischen Führungsdrahtes (4) und zum Anbringen an einem medizinischen Gerät, insbesondere an einem Endoskop, mit einem Anbringungsteil (2), durch welches der Führungsdraht (4) hindurchführbar ist, und einem mit dem Anbringungsteil (2) verbundenen und von diesem abstehenden Führungsdrahtträger (3), der den Führungsdraht (4) mittels einer Führungsdraht-Aufnahmeeinrichtung (9, 5, 7) festzuhalten gestattet, die ein erstes, den Führungsdraht (4) in Abstand von dem Anbringungsteil (2) in eine von seiner Durchführungsrichtung in dem Anbringungsteil (2) wegführende Richtung umleitendes und spannendes Führungsdraht-Umlenkglied (9) enthält, das einen auf einem flachen Anfangsbereich sich erhebenden, einseitig offenen Umlenk- und Spannbügel (9) aufweist, welcher den Führungsdraht (4) in einer Öffnung aufzunehmen und aus dieser wieder freizugeben gestattet, und an das sich in weiteren Abständen von dem Anbringungsteil (2) ein zusätzliches Führungsdraht-Umlenkelement (5) und ein noch weiteres Führungsdraht-Umlenkelement (7) anschließen,
**dadurch gekennzeichnet, dass** der Umlenk- und Spannbügel (9) zwei Bügelteile (9a, 9b) aufweist, die von dem Anfangsbereich des Führungsdrahtträgers (3) abstehen und zwischen denen ein die genannte Öffnung bildender Schlitz vorgesehen ist, der bei elastisch auseinander drückbaren Bügelteilen eine unterhalb der Querabmessung des Führungsdrahtes liegende Schlitzweite und bei starren Bügelteilen eine der Querabmessung des Führungsdrahtes (4) entsprechende Schlitzweite aufweist und der sich zwischen den zwei Bügelteilen (9a, 9b) in einer Position und/oder Ausrichtung außerhalb einer durch die Eigenelastizität des Führungsdrahts (4) gegebenen Führungsdraht-Aufweitungsbahn von dem Führungsdrahtträger (3) befindet, in der der Führungsdraht (4) versucht, sich in einer Richtung im Wesentlichen senkrecht zu seiner Verlaufsbahn aufzuweiten, indem er gegen die dem Führungsdrahtträger (3) zugewandte Unterseite des Umlenk- und Spannbügels (9) bzw. dessen Bügelteile (9a, 9b) auf seiner Aufweitungsbahn drückt.

2. Führungsdrahthalter (1) nach Anspruch 1,
**dadurch gekennzeichnet, dass** eines der beiden Bügelteile (9a, 9b) ein von dem Anfangsbereich (8) des Führungsdrahtträgers (3) abstehendes Tragglied (11a; 11b) und ein von diesem getragenes Oberteil (9a; 9b) mit einem gegenüber dem Querschnitt des Tragteiles (11a, 11b) ausladenden Querschnitt bzw. Körpervolumen aufweist und dass das andere Bügelteil (9b; 9a) lediglich ein Tragteil (11b) ist, das dem genannten Oberteil (9a; 9b) unter Bildung des Schlitzes (10) in Abstand benachbart ist.

3. Führungsdrahthalter (1) nach Anspruch 2,
**dadurch gekennzeichnet, dass** der Schlitz (10) lediglich im Bereich des ausladenden Querschnitts bzw. Körpervolumens des Oberteiles (9a; 9b) des einen Bügelteiles (9a; 9b) und dem das andere Bügelteil (9b; 9a) bildenden Tragteil (11b; 11a) vorgesehen ist.

4. Führungsdrahthalter (1) nach Anspruch 1, **dadurch**
**gekennzeichnet, dass** jedes der beiden Bügelteile (9a, 9b) ein von dem Anfangsbereich (8) des Führungsdrahtträgers (3) abstehendes Tragglied (11a; 11b) und ein von diesem getragenes Oberteil (9a; 9b) mit einem gegenüber dem Querschnitt des zugehörigen Tragteiles (11a; 11b) ausladenden Querschnitten bzw. Körpervolumen aufweist und dass die Oberteile (9a; 9b) der beiden Bügelteile unter Bildung des genannten Schlitzes (10) voneinander in Abstand vorgesehen sind.

5. Führungsdrahthalter (1) nach Anspruch 3 oder 4,
**dadurch gekennzeichnet, dass** das jeweilige Tragteil (11a; 11b), welches ein Oberteil (9a; 9b) trägt, mit seiner dem Schlitz (10) zugewandten Seite von diesem zurück versetzt

6. Führungsdrahthalter (1) nach einem der Ansprüche 2 bis 5, **dadurch**
**gekennzeichnet, dass** das jeweilige Oberteil (9a, 9b) mit seinem gegenüber dem Querschnitt seines Tragteiles (11a; 11b) ausladenden Querschnitt bzw. Körpervolumen rollen-, walzen-, kegel-, tonnen- oder kugelförmig aus gebildet ist.

7. Führungsdrahthalter (1) nach einem der Ansprüche 2 bis 6, **dadurch**
**gekennzeichnet, dass** das jeweilige Oberteil (9a, 9b) zu dem Schlitz (10) hin eine abgeflachte Fläche bildet.

8. Führungsdrahthalter (1) nach einem der Ansprüche 1 bis 7, **dadurch**
**gekennzeichnet, dass** der Schlitz (10) einen geradlinigen Verlauf aufweist.

9. Führungsdrahthalter (1) nach einem der Ansprüche 1 bis 8,
**dadurch gekennzeichnet, dass** der im mittleren Bereich des Umlenk- und Spannbügels (9) zwischen dessen beiden Bügelteilen (9a, 9b) vorgesehene Schlitz (10) unter einem Winkel α zwischen 5° bis 175° bezogen auf die Aufnahme- und Festhaltebahn des Führungsdrahts (4) auf dem Führungsdrahtträger (3) verläuft.

10. Führungsdrahthalter (1) nach einem der Ansprüche 1 bis 9,
**dadurch gekennzeichnet, dass** der Führungsdrahtträger (3) im Bereich der Aufnahme- und Festhaltebahn des Führungsdrahts (4) ein Material, wie Gummi oder Silikon aufweist, welches auf den daran anliegenden Führungsdraht (4) einen Reibwiderstand ausübt.

## Claims

1. Guide wire holder (1) for accommodation and holding in place of a medical guide wire (4) that possesses inherent elasticity, and for attachment to a medical device, particularly to an endoscope, having an attachment part (2) through which the guide wire (4) can be passed and a guide wire support (3) connected with the attachment part (2) and projecting away from the latter, which support permits the guide wire (4) to be held in place by means of a guide wire accommodation device (9, 5, 7), which device contains a first guide wire deflection member (9) that deflects and clamps the guide wire (4) at a distance from the attachment part (2), in a direction leading away from its passage direction in the attachment part (2), which member has a deflection and clamping bracket (9) that is open on one side and rises from a flat initial region, which bracket allows the guide wire (4) to be accommodated in an opening and released from the latter again, and is followed, at further distances from the attachment part (2), by an additional guide wire deflection element (5) and yet a further guide wire deflection element (7),
**characterized in that** the deflection and clamping bracket (9) has two bracket parts (9a, 9b), which project away from the initial region of the guide wire support (3) and between which a slot that forms the said opening is provided, which slot has a slot width that lies below the transverse dimension of the guide wire in the case of bracket parts that can be pressed elastically apart from one another, and a slot width that corresponds to the transverse dimension of the guide wire (4) in the case of rigid bracket parts, and which is situated between the two bracket parts (9a, 9b) in a position and/or alignment outside of a guide wire expansion path of the guide wire support (3), which path is determined by the inherent elasticity of the guide wire (4), in which position the guide wire (4) attempts to expand in a direction essentially perpendicular to its progressive path by pressing against the bottom side of the deflection and clamping bracket (9) or its bracket parts (9a, 9b) facing the guide wire support (3) on its expansion path.

2. Guide wire holder (1) according to claim 1,
**characterized in that** one of the two bracket parts (9a, 9b) has a support member (11a; 11b) that projects away from the initial region (8) of the guide wire support (3), and an upper part (9a; 9b) carried by this member, having a cross-section or body volume that projects away relative to the cross-section of the support part (11a, 11b), and that the other bracket part (9b; 9a) is merely a support part (11b), which is adjacent to the said upper part (9a; 9b) at a distance, whilst forming the slot (10).

3. Guide wire holder (1) according to claim 2,
**characterized in that** the slot (10) is provided only in the region of the projecting cross-section or body volume of the upper part (9a; 9b) of the one bracket part (9a; 9b) and the support part (11b; 11a) that forms the other bracket part (9b; 9a).

4. Guide wire holder (1) according to claim 1,
**characterized in that** each of the two bracket parts (9a, 9b) has a support member (11a; 11b) that projects away from the initial region (8) of the guide wire support (3), and an upper part (9a; 9b) supported by the latter, having a cross-section or body volume that projects relative to the cross-section of the related support part (11a; 11b), and that the upper parts (9a; 9b) of the two bracket parts are provided at a distance from one another, forming the said slot (10).

5. Guide wire holder (1) according to claim 3 or 4,
**characterized in that** the respective support part (11a; 11b), which supports an upper part (9a; 9b) with its side facing the slot (10) is set back from same.

6. Guide wire holder (1) according to any one of claims 2 or 5,
**characterized in that** the respective upper part (9a, 9b), with its cross-section or body volume that projects relative to the cross-section of its support part (11a; 11b), is configured in the shape of a roller, roll, cone, barrel or sphere.

7. Guide wire holder (1) according to any one of claims 2 to 6,
**characterized in that** the respective upper part (9a, 9b) forms a flattened surface toward the slot (10).

8. Guide wire holder (1) according to any one of claims 1 to 7,
**characterized in that** the slot (10) has a straight-line progression.

9. Guide wire holder (1) according to any one of claims 1 to 8,
**characterized in that** the slot (10) provided in the central region of the deflection and clamping bracket (9), between its two bracket parts (9a, 9b), runs at an angle α between 5° and 175° with reference to the accommodation and holding in place path of the guide wire (4) on the guide wire support (3).

10. Guide wire holder (1) according to any one of claims 1 to 9,
**characterized in that** the guide wire support (3) has a material such as rubber or silicone in the region of the accommodation and holding in place path of the guide wire (4), which material exerts a friction resistance on the guide wire (4) that lies against it.

## Revendications

1. Support de fil de guidage (1), destiné à loger et retenir un fil de guidage médical (4), possédant une élasticité propre et à être apposé sur un appareil médical, en particulier sur un endoscope, avec une partie pose (2), à travers laquelle peut être passé le fil de guidage (4) et un support de fil de guidage (3), relié à la partie pose (2) et à distance de celle-ci, qui permet de retenir le fil de guidage (4) au moyen d'un dispositif de logement de fil de guidage (9, 5, 7), qui renferme un premier élément de renvoi de fil de guidage (9), qui dévie et serre le fil de guidage (4) à l'écart de la partie pose (2) dans une direction qui s'éloigne de sa direction de passage dans la partie pose (2), qui présente un étrier de renvoi et de serrage (9), qui s'élève sur une zone initiale plate, ouverte d'un côté, laquelle permet de loger le fil de guidage (4) dans une ouverture et de le dégager à nouveau de celle-ci et auquel se raccordent un élément de renvoi de fil de guidage (5) supplémentaire et encore un autre élément de renvoi de fil de guidage (7), suivant d'autres intervalles, par rapport à la partie pose (2),
**caractérisé en ce que**
l'étrier de renvoi et de serrage (9) présente deux parties formant étrier (9a, 9b), qui s'écartent de la zone initiale du support de fil de guidage (3) et entre lesquelles est prévue une fente, qui forme l'ouverture nommée, qui présente, dans des parties formant étrier, qui peuvent se repousser élastiquement l'une de l'autre, une largeur de fente, qui se trouve au-dessous de la dimension transversale du fil de guidage et, dans des parties formant étrier rigides, une largeur de fente, qui correspond à la dimension transversale du fil de guidage (4) et qui se trouve, entre les deux parties formant étrier (9a, 9b), dans une position et / ou suivant une orientation à l'extérieur d'une voie d'élargissement du fil de guidage, apportée par l'élasticité propre du fil de guidage (4), par rapport au support du fil de guidage (3), dans laquelle le fil de guidage (4) tente de s'élargir dans une direction essentiellement verticalement par rapport à sa voie d'évolution, en appuyant sur sa voie d'élargissement, contre la face inférieure de l'étrier de renvoi et de serrage (9) ou de ses parties formant étrier (9a, 9b), tournée vers le support du fil de guidage (3).

2. Support de fil de guidage (1) selon la revendication 1,
**caractérisé en ce que**
l'une des deux parties formant étrier (9a, 9b) présente un élément formant support (11a ; 11b), s'écartant de la zone initiale (8) du support de fil de guidage (3) et une partie supérieure (9a ; 9b), supportée par celui-ci, avec une section ou un volume corporel en surplomb par rapport à la section de la partie formant support (11a ; 11b) et que l'autre partie formant étrier (9b ; 9a) n'est qu'une partie formant support (11b), qui est proche de la partie supérieure (9a, 9b) nommée, pour former la fente (10) à l'écart.

3. Support de fil de guidage (1) selon la revendication 2,
**caractérisé en ce que**
la fente (10) n'est prévue que dans la zone de la section ou du volume corporel en surplomb de la partie supérieure (9a ; 9b) d'une partie formant étrier (9a ; 9b) et de celle de la partie formant support (11b ; 11a), formant l'autre partie formant étrier (9b ; 9a).

4. Support de fil de guidage (1) selon la revendication 1,
**caractérisé en ce que**
chacune des deux parties formant étrier (9a, 9b) présente un élément formant support (11a ; 11b), s'écartant de la zone initiale (8) du support de fil de guidage (3) et une partie supérieure (9a ; 9b), supportée par celui-ci, avec des sections ou des volumes corporels en surplomb par rapport à la section de la partie formant support (11a ; 11b) associée et que les parties supérieures (9a ; 9b) des deux parties formant étrier sont prévues écartées l'une de l'autre, pour former la fente (10) nommée.

5. Support de fil de guidage (1) selon la revendication 3 ou 4,
**caractérisé en ce que**
la partie formant support (11a ; 11b) respective, qui supporte une partie supérieure (9a ; 9b), est décalée de celle-ci en arrière, avec sa face tournée vers la fente (10).

6. Support de fil de guidage (1) selon l'une des revendications 2 à 5,
**caractérisé en ce que**
la partie supérieure (9a, 9b) respective est formée, avec sa section ou son volume corporel, en surplomb par rapport à la section de sa partie formant support (11a ; 11b), en forme de rouleau, de cylindre, de cône, de barillet ou de sphère.

7. Support de fil de guidage (1) selon l'une des revendications 2 à 6,
**caractérisé en ce que**
la partie supérieure (9a, 9b) respective forme une surface aplatie par rapport à la fente (10).

8. Support de fil de guidage (1) selon l'une des revendications 1 à 7,
**caractérisé en ce que**
la fente (10) présente un parcours rectiligne.

9. Support de fil de guidage (1) selon l'une des revendications 1 à 8,
**caractérisé en ce que**
la fente (10), prévue dans la zone centrale de l'étrier de renvoi et de serrage (9) entre ses deux parties formant étrier (9a, 9b), évolue suivant un angle α entre 5° à 175° par rapport à la voie de logement et de retenue du fil de guidage (4) sur le support de fil de guidage (3).

10. Support de fil de guidage (1) selon l'une des revendications 1 à 9,
**caractérisé en ce que**
le support de fil de guidage (3) présente, dans la zone de la voie de logement et de retenue du fil de guidage (4), un matériau, tel que du caoutchouc ou du silicone, lequel exerce sur le fil de guidage (4) qui y adhère, une résistance à la friction.
